# EUROPEAN PATENT SPECIFICATION

(11) **EP 3 572 810 B1**
(45) Date of publication and mention of the grant of the patent: **30.10.2024**
(21) Application number: 17840470.3
(22) Date of filing: 20.01.2017
(51) Int. Cl.: G01N 33/535, G01N 21/76

(54) **LABELLED COMPLEX AND PREPARATION METHOD THEREFOR, AND KIT, USE AND DETECTION SYSTEM THEREOF**
MARKIERTER KOMPLEX UND HERSTELLUNGSVERFAHREN DAFÜR SOWIE KIT, VERWENDUNG UND DETEKTIONSSYSTEM DAFÜR
COMPLEXE MARQUÉ ET PROCÉDÉ DE PRÉPARATION CORRESPONDANT ET KIT ET SYSTÈME D'UTILISATION ET DE DÉTECTION CORRESPONDANT

(43) Date of publication of application: 27.11.2019
(73) Proprietor: Shenzhen New Industries Biomedical Engineering Co., Ltd., Shenzhen, Guangdong 518122 (CN)
(72) Inventor: RAO, Wei, Shenzhen Guangdong 518122 (CN); FANG, Zhonggang, Shenzhen Guangdong 518122 (CN); YUAN, Jinyun, Shenzhen Guangdong 518122 (CN); WANG, Yanmei, Shenzhen Guangdong 518122 (CN); LI, Tinghua, Shenzhen Guangdong 518122 (CN); WANG, Xiaoli, Shenzhen Guangdong 518122 (CN)
(74) Representative: Zacco GmbH
(86) International application number: PCT/CN2017/071968
(87) International publication number: WO 2018/133038

(56) References cited:
- EP-A1- 1 845 378
- WO-A1-2010/099479
- WO-A1-2014/059274
- WO-A1-2016/004613
- WO-A1-2016/127322
- CN-A- 101 051 047
- CN-A- 101 051 047
- CN-A- 102 333 885
- US-A1- 2002 106 636
- US-A1- 2014 272 933
- US-A1- 2015 285 827
- WANG YANHU ET AL: "Chemiluminescence excited photoelectrochemical competitive immunosensing lab-on-paper device using an integrated paper supercapacitor for signal amplication", SENSORS AND ACTUATORS B: CHEMICAL, ELSEVIER BV, NL, vol. 208, 21 November 2014 (2014-11-21), pages 546 - 553, XP029109493, ISSN: 0925-4005, DOI: 10.1016/J.SNB.2014.11.088
- TIAN DAYONG ET AL: "Synthesis of N-(aminobutyl)-N-(ethylisoluminol) functionalized gold nanomaterials for chemiluminescent bio-probe", CHEMICAL COMMUNICATIONS, vol. 47, no. 17, 1 January 2011 (2011-01-01), UK, pages 4959, XP093132297, ISSN: 1359-7345, DOI: 10.1039/c1cc10435g

## Description

### Technical Field

The present disclosure relates to the field of in-vitro diagnostic reagents, and in particular to a labeled complex, the preparation method thereof, a kit containing the same, the application of the kit, and a detection system comprising the kit.

### Background

At present, methods used for clinical immunodetection mainly include Enzyme-Linked Immuno Sorbent Assay (ELISA), Western Blot (WB), Immune Colloidal Gold Technique (GICT), Radio Immuno Precipitation Assay (RIPA) and Chemiluminescence immunoassay (CLIA). ELISA and CLIA are suitable for large-scale clinical preliminary screening but are likely to present a false positive or false negative detection result because of the instability or inadequate performance of the components of a reagent. Therefore, it is necessary to increase the sensitivity and stability of a reagent.

ELISA, a detection technique currently widely used for clinical blood screening, is more complicated in test procedure and more likely to present a false positive or false negative result than CLIA, which is advantaged in full automatic operation and high sensitivity. Quick detection test strips represented by colloidal gold or latex particles appear at home and abroad in recent years, but because their test results are visually observed by naked eyes and are easily affected by the subjective judgment of the observers, these quick detection test strips are low in sensitivity and not high in test accuracy.

As one of confirmation methods currently used for clinical tests, WB provides an accurate and reliable result but is technically difficult and can only be produced by few companies worldwide, its high usage cost makes it only currently suitable for suspicious specimen confirmation and seldom for screening.

With advantages of high sensitivity, wide linear range, excellent specificity, simple equipment and device operations and so on, CLIA has been successfully used in fields including bioscience, food science, clinical medicine and environmental monitoring and has become an effective microanalysis and trace analysis technique in the field of modern analysis. According to luminescence mechanism, luminescent substances for chemiluminescence reaction are mainly classified into: luminal and derivates thereof, lucigenin, peroxyoxalate, and the like. Derivates of luminal mainly include isoluminol, 4-aminohexyl-N ethylisoluminol and the like. Luminal can be oxidized by some oxidizing agents under an alkaline condition to produce a chemiluminescence reaction to generate chemiluminescence having a maximum emission wavelength of 425nm. Lucigenin (bis-N-methylacridinium nitrate) itself generates weak chemiluminescence in an alkaline environment, but when added with hydrogen peroxide, lucigenin is oxidized and strongly enhanced in chemiluminescence intensity to emit light of 420-500nm and therefore has a high luminous efficiency. The chemiluminescence reaction of peroxalate, which involves the reaction between hydrogen peroxide and aryl oxalates, is the most effective non-biological chemiluminescence reaction. Chemiluminiscence reagents of luminals and acridinium esters are the most common chemiluminescence labeling reagents; common enzyme makers include Horse Radish Peroxidase (HRP) and Alkaline Phosphatase (ALP), which both have their own chemiluminescent substrate; however, in actual applications, the main electrochemical luminescence system is a ruthenium bipyridyl [Ru(bpy)3]²⁺ system.

At present, double-antigen (double-antibody) sandwich method is extensively used in immunodetection. The principle of double-antigen (double-antibody) sandwich method resides in that a solid phase material is coated by a first antigen (antibody), a sample to be detected is added, and a second antigen (antibody) with a marker is added for test. If the sample to be detected contains a substance to be detected, then a sandwich structure of "coating antigen (antibody)-antibody (antigen)-labeled antigen (antibody)" is formed, and a result is determined by detecting the signal of the marker. One of the key factors for applying the double-antigen (double-antibody) sandwich method lies in the quality of the labeled antigen because the activity of the labeled antigen is critical to the sensitivity and stability of an immunodetection. A highly active labeled antigen needs to meet two conditions: 1: the markers combined with each antigen in the labeled antigen should be as many as possible; 2: a labeling process should cause as little loss to the immunological activity of the antigen as possible. The activity of many labeled antigens formed by coupling antigens with markers is severely lowered. The typical solution to this is fusing and expressing a segment of fusion protein on an antigen to form an expression form of a chimeric antigen of fusion protein-antigen, a recombinant antigen expressed using this method is better in solubility and expression quantity and therefore has a superb labeling performance. Nonetheless, the application of fusion protein has, to some extent, shortages, for example, the adoption of a prokaryotic expression system usually gives birth to an inclusion body, making it occur that a labeled antigen is likely to present a false positive result.

In Chinese Patent Publication CN1888901A, which proposes a reagent for direct chemiluminescence using magnetic separation and test method thereof, an isoluminol derivate is used as a luminescence marker, the monoclonal antibody of the isoluminol chemiluminescence marker is linked with a monoclonal antibody after the isoluminol chemiluminescence marker is linked with thiocarbonyl chloride or hydroxy-butanedioic acid. This patent application gives no concern to improve the stability and sensitivity of a luminescence marker for a recombinant antigen through a secondary labeling.

In Chinese Patent Publication CN100582780A, which proposes "an assay of hepatitis C virus total antigen using double-antigen sandwich method of indirect small molecule labeling", a reagent marks a HCV antigen with a small molecule, and the anti-small molecule antibody or another substance that can be bound with small molecules is labeled with a signal generation substance, and the HCV antigen coats a solid phase material. The anti-HCV antibody in a specimen can react with the HCV antigen on the surface of a solid phase material and an HCV antigen labeled with a small molecule to form a double-antigen sandwich complex, and then the small molecule reacts with a signal report molecule so that the formed complex carries a detectable signal generation substance. The use of small molecules proposed in this patent application helps to keep the activity of an antigen and has a signal amplification function and therefore improves the sensitivity and specificity of HCV antibody detection. This patent application requires a suitable small molecule substance as well as an anti-small molecule antibody or a substance that can be bound with small molecules, moreover, this patent application also requires a research on the stability of an antigen labeled with a small molecule and that of an anti-small molecule antibody labeled with a signal generation substance.

It has been proposed in PCT Patent Application EP20050714754 and in Chinese Patent Publication CN101363848A that "double-antigen sandwich method for detecting antibody by indirectly labeling nano-particle and kit thereof" , in which the labeling of an antigen labeled by a nano-particle marker is an indirect labeling completed through binding a tag on the antigen with a ligand on the nano-particle marker that can specifically recognize the tag, wherein the labeled antigen is a genetically engineered recombinant antigen. With the adoption of this indirect labeling method, the sensitivity and specificity of a double-antigen sandwich method for detecting an antibody with nano-particles can be improved significantly. This patent application requires the labeling of a marker protein on a recombinant antigen being expressed, a ligand capable of specifically recognizing the tag, and the labeling of the ligand. As a result, two kit components are needed; moreover, it is needed to research the optimal reaction ratio of the two components. Besides, this patent application also fails to take into consideration the stability of a marker protein or a marker protein ligand marker.

A reagent and a method for stabilizing alkalin phosphatase or marker thereof are disclosed in Chinese Patent CN102115737B, a diluent capable of maintaining the high stability of an enzyme marker solution is disclosed in Chinese Patent CN101561432B, the two patents are both directed at improving the stability of a marker by optimizing the components of a marker diluent. By optimizing ion strengths and pH values of protective proteins and a solution in the diluent and increasing polyhydric alcohol, polysaccharide surfactant and protease inhibitor, alkaline phosphatase or its marker almost suffers no loss after a two-week accelerated breakdown test at 37°C. A diluent consisting of 0.05M Phosphate-buffered saline (PBS) whose pH is 7.2, protective proteins, compound amino acid, polysaccharide, an aloe extract, rat serum, potassium gluconate, sodium caprylate, Tween and a preservative can preserve an HRP marker for more than one week at 37°C. Appropriate diluent components are the precondition of guaranteeing the stability of a labeled complex; however, it is needed to change the labeling process of a labeled complex when optimizing the components of a diluent is inadequate for changing stability.

A paclitaxel-BSA labeled with a detectable label, such as fluorescent moiety, antibody with colloidal gold, was disclosed by US 2015 0285827A1.

CN101051047A disclosed a immune analytical method of chemilumi-nescence enzyme, and disclosed a HRP-labeled specific binding substance, such as mouse-IgG, a chemilumi-nescence substrate-labeled specific binding substrate, and a sample to be analylized. The sample is bound by both the HRP-labeled substance and the chemilumi-nescence substrate-labeled specific binding substrate.

WO 2014 059274 A1 disclosed a peptide(such as SEQ ID NO:72) coupled with BSA, then conjugated to a detectable label, such as colloidal gold, or other labels, such as fluorescent, or enzyme labels, metal particles.

US 2002 0106636 A1 disclosed a monoclonal antibody which may be used in the detection of HIV, the HIV may be a recombinant protein.

US 2014 0272933 A1 disclosed a immunoassay for combined detection of HCV antigen and HCV antibody in a test sample, and also disclosed a biotinylated anti-HCV antibody for the capture of an HCV antigen present in the test sample, and a detectably labeled HCV antigen for binding to anti-HCV antibody captured by the biotinylated HCV antigen.

Further relevant prior art is disclosed in non-patent literatures "Chemiluminescence excited photoelectrochemical competitive immunosensing lab-on-paper device using an integrated paper supercapacitor for signal amplication", SENSORS AND ACTUATORS B: CHEMICAL, ELSEVIER BV, NL, vol. 208, 21 November 2014, pages 546-553, XP029109493, and "Synthesis of N-(aminobutyl)-N-(ethylisoluminol) functionalized gold nanomaterials for chemiluminescent bio-probe", Chemical Communications, vol. 47, no. 17, 1 January 2011, page 4959, XP093132297.

Existing solutions mainly have the following technical problems:
1) Most of existing methods for labeling an antigen or antibody are direct labeling, however, the immunological activity of some antigens is lowered after these antigens are directly labeled with a detected signal substance, and consequentially, the stability and sensitivity of a marker is impaired;
2) Although the sensitivity of a labeled antigen can be improved by using a micromolecule or marker protein indirect labeling method, it is needed to look for a proper micromolecule or marker protein and a corresponding recognizable ligand, moreover, the components of a reagent are increased, and the reagent is consequentially complicated;
3) The expression of a chimeric antigen as fusion protein-antigen provides a recombinant antigen a higher solubility and a better labeling performance, however, the application of fusion protein has, to some extent, shortages, for example, the adoption of a prokaryotic expression system usually gives birth to an inclusion body, making it occur that a labeled antigen is likely to present a false positive result.

### Summary

The invention is set out in the appended set of claims. The present disclosure is intended to provide a labeled complex, the preparation method thereof, a kit containing the same, the application of the kit and a detection system comprising the kit, so as to improve the sensitivity of the labeled complex.

To this end, in accordance with an aspect of the present disclosure, a labeled complex is provided, comprising:
a labeled-complex intermediate, wherein the labeled-complex intermediate comprises an antigen; and a marker protein wherein the marker protein has a functional group on the surface thereof, and the functional group is one or more of carboxyl, amino, hydroxyl, sulfydryl, aldehyde group, glycosyl and imidazolyl, and the functional group is covalently-coupled with free amino, carboxyl, sulfydryl, aldehyde group or glycosyl on the antigen, and wherein the marker protein is horse radish peroxidase, alkaline phosphatase, acid phosphatase, glucose oxidase or β-galactosidase; and
a signal generation substance covalently-coupled with the marker protein of the labeled-complex intermediate to form the labeled complex, wherein the marker protein and the signal generation substance are different substances .

Further, the antigen is a recombinant antigen or a natural antigen.

Further, the antigen is a recombinant HIV-1 antigen, a recombinant HIV-2 antigen, a hepatitis A virus antigen, a hepatitis B virus antigen, a hepatitis C virus antigen, a hepatitis D virus antigen, a hepatitis E virus antigen, a hepatitis G virus antigen, a human T lymphocyte virus antigen, a treponema pallidum antigen, a helicobacter pylori antigen or a human papilloma virus antigen, and preferably, the antigen is the recombinant HIV-1 antigen or the recombinant HIV-2 antigen.

Further, the signal generation substance is luminal, isoluminol and a derivate thereof, alkaline phosphatase, horse radish peroxidase, a fluorescent substance, a rare earth ion and a chelate ligand thereof, acridinium ester and a derivate thereof, or tris (bipyridine) ruthenium, and preferably, the isoluminol derivate is N-(4-aminobutyl)-N-ethylisoluminol.

In accordance with another aspect of the present disclosure, a method for preparing the labeled complex is provided, comprising the following steps of: performing a covalent-coupling reaction between a marker protein having a functional group on the surface thereof and an antigen to obtain a labeled complex intermediate; and performing a covalent-coupling reaction between a signal generation substance and the marker protein of the labeled-complex intermediate to obtain the labeled complex; wherein the functional group is one or more of carboxyl, amino, hydroxyl, sulfydryl, aldehyde group, glycosyl and imidazolyl, and the functional group is covalently-coupled with free amino, carboxyl, sulfydryl, aldehyde group or glycosyl on the antigen, and the marker protein is horse radish peroxidase, alkaline phosphatase, acid phosphatase, glucose oxidase or β-galactosidase, the marker protein and the signal generation substance are different substances.

Further, the method used for the covalent-coupling reaction is a mixed anhydride method, a carbodiimide method, a glutaraldehyde method, a glutaric anhydride method, a diazotization method, a succinic anhydride method, a carbonyldiimidazole method or a sodium periodate method, and preferably, a cross-linking agent used in the covalent-coupling reaction is a homobifunctional amino reactive protein cross-linking agent based on NHS-ester and an imidoate reactive group, a sulfydryl-saccharide cross-linking agent based on maleimide and a hydrazide reactive group, a homobifunctional amino reactive protein cross-linking agent based on maleimide or a pyridine disulfydryl reactive group, a heterobifunctional protein cross-linking agent suitable for linking a protein with a primary amine and a sulfydryl of an another molecule, a carbodiimide cross-linking agent suitable for linking carboxyl with primary amine, a heterobifunctional cross-linking agent included in photoreactive cross-linking agents, a chemoselectivity cross-linking agent or a bifunctional cross-linking agent.

Further, a molar ratio of the marker protein to the antigen is 1:1 - 3:1 when performing the covalent-coupling reaction between the marker protein and the antigen.

Further, a buffer solution used in the covalent-coupling reaction is a PBS buffer solution whose pH is 4.0-6.5, a carbonate buffer solution whose pH is 8.0-9.8, a glutaraldehyde solution whose concentration is 0.5%-5% and pH is 7.0-8.0, or an MES buffer solution whose pH is 4.0-6.0.

Further, after prepared, the labeled-complex intermediate or the labeled complex is placed in a stock solution for storage, and the stock solution comprises: 0.2-0.5g/L potassium dihydrogen phosphate, 3.0-6.0g/L disodium hydrogen phosphate, 4.0-8.0g/L sodium chloride, 0.5-1.5g/L BSA, 0.25-0.5g/L sodium azide and 0.25-0.5mL/L Tween-20.

In accordance with still another aspect of the present disclosure, a detection kit is provided. The kit includes any one of the foregoing labeled complexes or a labeled complex prepared using any one of the foregoing labeled complex preparation methods.

Further, the kit also includes: a magnetic particle solution coated with an antigen; or a magnetic particle solution coated with streptavidin and a biotinylated antigen solution; or a magnetic particle solution coated with an anti-fluorescein isothiocyanate antibody and an antigen solution labeled with fluorescein isothiocyanate.

Further, the detection kit is a kit for detecting a HIV-1 antibody, which contains the labeled complex of a recombinant HIV-1 antigen; and preferably, the kit specifically includes: a magnetic particle solution coated with a recombinant HIV-1 antigen and the labeled complex solution of the recombinant HIV-1 antigen; or a magnetic particle solution coated with streptavidin, the labeled complex solution of a recombinant HIV-1 antigen and a biotinylated recombinant HIV-1 antigen solution; or a magnetic particle solution coated with an anti-fluorescein isothiocyanate antibody and a recombinant HIV-1 antigen solution labeled with fluorescein isothiocyanate; preferably, a concentration of the recombinant HIV-1 antigen in the magnetic particle solution coated with the recombinant HIV-1 antigen is 10-200µg/L; preferably, a concentration of the magnetic particles is 0.25-1.25mg/mL; preferably, a concentration of the recombinant HIV-1 antigen in the labeled complex solution of the recombinant HIV-1 antigen is 10-200µg/L; preferably, a concentration of the labeled complex of the recombinant HIV-1 antigen is 0.1-1mg/L; preferably, a concentration of the recombinant HIV-1 antigen in the biotinylated recombinant HIV-1 antigen solution is 10-200µg/L, and a concentration of biotin is 0.1-1mg/L; and preferably, a concentration of the recombinant HIV-1 antigen in the recombinant HIV-1 antigen solution labeled with fluorescein isothiocyanate is 10-200µg/L, and a concentration of the fluorescein isothiocyanate is 0.1-1 mg/L.

In accordance with yet another aspect of the present disclosure, the application of the kit in immunodetection is provided, wherein a marker protein and a detected protein are non-homologous proteins.

Further, the kit is used for detecting an HIV-1 antibody, an HIV-2 antibody, a hepatitis A virus, a hepatitis B virus, a hepatitis C virus, a hepatitis D virus, a hepatitis E virus, a hepatitis G virus, a human T lymphocyte virus, a treponema pallidum antibody, a helicobacter pylori antibody or a human papilloma virus antibody.

Further, the kit is applied to a semi-automatic or fully-automatic immune analyzer.

In accordance with yet still another aspect of the present disclosure, a detection system is provided. The detection system includes any one of the foregoing kits and a semi-automatic or fully-automatic immune analyzer. By applying the technical solutions provided herein to couple a marker protein with a functional group of an antigen, the conformation of the antigen is maintained, or changed without covering an epitope, thus keeping the immune reaactivity of the antigen. Moreover, by labeling an antigen with marker proteins, one antigen can carry a plurality of marker proteins, each of which is bound with a plurality of signal generation substances during a secondary signal generation substance labeling process to generate an effective signal amplification effect, thus further to improve the detection sensitivity.

### Brief Description of the Drawings

The accompanying drawings described herein are provided for a better understanding of the present disclosure and constitute one part of the present disclosure, and the exemplary embodiments of the present disclosure and description thereof are illustrative of the present disclosure but are not to be construed as limiting the present disclosure. In the accompanying drawings:

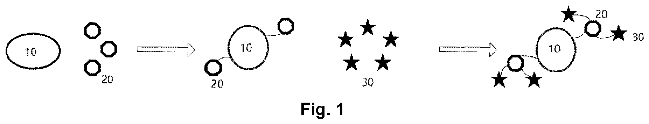

Fig. 1 is a schematic diagram illustrating the preparation process and the structure of a labeled complex according to an embodiment of the present disclosure.

### Detailed Description of the Embodiments

It should be noted that embodiments of the present disclosure and the features thereof can be combined with each other if no conflict is caused. The present disclosure is described below in detail with reference to accompanying drawings when read in conjunction with embodiments.

The abbreviations and the terms involved herein are explained as follows:
HIV: Human Immunodeficiency Virus; AIDS: Acquired Immune Deficiency Syndrome; CLIA: Chemiluminescence Immune Assay; BSA: Bovine Serum Albumin; NaNs : sodium azide; ABEI: N-(4-Aminobutyl)-N-Ethylisoluminol; FITC: Fluorescein Isothiocyanate; Biotin; SA: Streptavidin; HRP: Horse Radish Peroxidase; AE: Acridinium Ester; ALP: Alkaline Phosphatase; Anti-FIFC antibody: Anti-fluorescein isothiocyanate antibody; ELISA: Enzyme Linked Immunosorbent Assay; WB: Western Blot; GICT: Immune Colloidal Gold Technique; and RIPA: Radio Immunoprecipitation Assay.
Labeling: the term 'labeling', as used herein, refers to the coupling of a ligand with a marker.
Marker: the term 'marker', as used herein, refers to a substance detectable in immunodetection or a labeled complex containing the marker.
Fusion protein: the term 'fusion protein', as used herein, refers to a protein which is expressed with a labeled antigen as a fusion antigen, and the fusion antigen can be coupled with a marker to detect a target antibody.
Marker protein: the term 'marker protein', as used herein, refers to a protein used for labeling an antigen primarily.
Labeled antigen: the term 'labeled antigen', as used herein, refers to an antigen bound with a target antibody to be detected in an immunodetection.
Indirect labeling: the term 'indirect labeling' as used herein, refers to the direct labeling of a marker with a ligand through the specific recognition of a tag on a labeled antigen and a corresponding ligand, but not the direct bonding of the labeled antigen with the marker.
Secondary labeling: the term 'secondary labeling', as used herein, refers to labeling, after a labeled protein is labeled with a protein through a primary labeling, a labeled complex secondarily with a detectable signal substance.
Ligand: the term 'ligand', as used herein, refers to a molecule, for example, antibody, an avidin and the like, which is specifically recognizable to a tag.
Tag: the term 'tag', as used herein, includes, but is not limited to, polypeptide or protein, biotin or combinations of biotins and polypeptides or proteins, the polypeptide or protein mentioned here is polypeptide or protein different from any segment of the coating antigen of a kit.
Cross-linking agent: the term 'cross-linking agent', as used herein, refers generally to a kind of small molecule compounds having two or more reactive ends specific to special groups (-NH₂,-COOH,-HS and so on), the two or more reactive ends can be separately coupled with two or more molecules to be bound with these molecules.
Biotin: it may be hereinafter abbreviated as Bio.
Streptavidin: it may be hereinafter abbreviated as avidin or SA.

The term 'signal generation substance' or "marker", as used herein, refers to a part which is linked with a specifically bound partner, for example, an antigen or an analyte, so that the specifically bound partner (e.g. an antigen and an analyte) is detectable, moreover, a specifically bound partner (e.g. an antigen or an analyte) labeled in this way is called 'detectable'. The labeling may generate a signal detectable to a visual device or an apparatus. Various markers include a signal generation substance such as a chromogen, a fluorescent compound, a chemiluminescent compound and a radioactive compound. Representative examples of the marker include a light generating part (e.g. an acridinium ester compound) and a fluorescence generating part (e.g. a fluorescein).

In the prior art, after an antigen is directly labeled, the stability and sensitivity of a labeled complex is not sufficient, and the requirements on the performance of a reagent cannot be met by independently optimizing the compositions of a buffer solution, and indirect labeling requires a suitable small molecule or marker protein and a corresponding recognizable ligand. At the same time, making it more difficult to express a recombinant antigen and increasing the components of a reagent.

To increase the sensitivity of a labeled complex, the present disclosure provides the following technical solutions: as shown in Fig. 1, performing a primary labeling on a marker protein 20 having a functional group on the surface thereof and an antigen 10 to obtain a labeled complex intermediate; performing a secondary labeling treatment on a signal generation substance 30 and The labeled complex intermediate, and then performing a separation and purification treatment to remove unbound substances, thereby obtaining a final labeled complex.

According to a typical embodiment of the present disclosure, the labeled complex is provided. The labeled complex includes an antigen; a marker protein coupled with the antigen to form a labeled complex intermediate; and a signal generation substance coupled with the labeled-complex intermediate to form the labeled complex.

Why the present disclosure can achieve technical effects is analyzed below: protein denaturation refers to when a protein is affected by physical or chemical factors, the secondary, tertiary and quaternary structures within their molecules will change, resulting in the loss of biological functions or changes in physical or chemical properties. Protein denaturation caused by temperature is mainly destabilization of non-covalent interactions. Thus, when some antigen-labeled labeled complexes are under conditions of high temperature, the conformation of the antigen is changed, leading to the antigen epitope being covered and leading to the decrease or loss of immunological reactivity of the antigen. By coupling a marker protein with a functional group of an antigen, the conformation of the antigen is maintained, or changed without covering an epitope, thus keeping the immune reactivity of the antigen. By labeling an antigen with marker proteins, one antigen can carry a plurality of marker proteins, each of which is bound with a plurality of signal generation substances during a secondary signal generation substance labeling process to generate an effective signal amplification effect, thus further to improve the detection sensitivity.

Further, the antigen is a recombinant antigen or a natural antigen; preferably, the antigen includes, but is not limited to, a recombinant HIV-1 antigen, a recombinant HIV-2 antigen, a hepatitis A virus antigen, a hepatitis B virus antigen, a hepatitis C virus antigen, a hepatitis D virus antigen, a hepatitis E virus antigen, a hepatitis G virus antigen, a human T lymphocyte virus antigen, a treponema pallidum antigen, a helicobacter pylori antigen or a human papilloma virus antigen, and preferably, the antigen is the recombinant HIV-1 antigen or the recombinant HIV-2 antigen.

Theoretically, any protein can be used as the marker protein of the present disclosure, preferably, the marker protein has the following properties: 1) with a -COOH, -NH₂, -OH, -SH, -NH₂, -CHO, -C=O, an imidazolyl and other chemically active functional group on its surface in order to couple to the free amino, carboxyl, mercapto, aldehyde or carbonyl groups on the antigen; 2) with the capacity of coupling sufficient molecules; 3) to prevent the occurrence of non-specific binding, the marker protein should be a protein non-homologous to the test object; and 4) the marker protein should be stable enough, cheap and easy to get.

Therefore, the marker protein of the invention is horse radish peroxidase, alkaline phosphatase, acid phosphatase, glucose oxidase or β-galactosidase.

The "signal generation substance" includes, but is not limited to, a chemiluminescent substance such as luminal, isoluminol and a derivate thereof, alkaline phosphatase, horse radish peroxidase, a fluorescent substance, a rare earth ion and a chelate ligand thereof, acridinium ester and a derivate thereof, or tris (bipyridine) ruthenium, and preferably, the isoluminol derivate is N-(4-aminobutyl)-N-ethylisoluminol (hereinafter referred to as ABEI for short).

According to the invention, the marker protein and the signal generation substance are different from substances, while the secondary labeling is performed because the primary labeling is inadequate in improving stability and sensitivity.

In accordance with another aspect of the present invention a method for preparing the labeled complex of any one of claims 1-3 is provided, comprising the following steps of: performing a covalent-coupling reaction between a marker protein having a functional group on the surface thereof and an antigen to obtain a labeled-complex intermediate; and performing a covalent-coupling reaction between a signal generation substance and the marker protein of the labeled-complex intermediate to obtain the labeled complex; wherein the functional group is one or more of carboxyl, amino, hydroxyl, sulfydryl, aldehyde group, glycosyl and imidazolyl, and the functional group is covalently-coupled with free amino, carboxyl, sulfydryl, aldehyde group or glycosyl on the antigen, and wherein the marker protein is horseradish peroxidase, alkaline phosphatase, acid phosphatase, glucose oxidase or β-galactosidase; wherein the marker protein and the signal generation substance are different substances.

The functional group carried on the surface of the marker protein includes, but is not limited to: -COOH, -NH₂, -OH, -SH, -CHO, -C=O and an imidazolyl, and the functional group is coupled with free -NH₂, -COOH, -SH, -CHO or carbonyl group on an antigen (because it is specified herein that the functional group carried on the surface of the marker protein is coupled with free -NH₂, -COOH, -SH, -CHO or carbonyl group on an antigen, those of ordinary skill in the art can clearly rule out the situation that the functional group on the marker protein cannot be coupled with a free group on an antigen when they are the same groups). A treatment is performed after the coupling reaction to remove unbound substances and other substances, wherein the treatment includes, but is not limited to: dialysis, ultrafiltration, ion-exchange chromatography, gel filtration, salt in and salting out, isoelectric point precipitation, affinity chromatography, and so on, and then the obtained labeled-complex intermediate is used or dispersed in a stock solution for storage.

According to a typical embodiment of the present disclosure, the preparation method specifically includes the following steps: A. performing a coupling reaction between a marker protein and an antigen by selecting a proper method based on functional groups to be coupled; B: removing unbound substances to obtain a labeled complex intermediate; C: performing a coupling reaction between a signal generation substance and the labeled-complex intermediate by selecting a proper method based on functional groups to be coupled; and D: removing unbound substances to obtain a final labeled complex.

To couple the signal generation substance with the labeled-complex intermediate by selecting a proper method based on functional groups to be coupled, and the method adopted for coupling reaction includes, but is not limited to: a mixed anhydride method, a carbodiimide method, a glutaraldehyde method, a glutaric anhydride method, a diazotization method, a succinic anhydride method, a carbonyl-diimidazole method or a sodium-periodate method.

The cross-linking agent used in the coupling reaction includes, but is not limited to:
1) An amino-amino cross-linking agent: a homobifunctional amino reactive protein cross-linking agent based on NHS-ester and an imidoate reactive group, for example, Di(N-succinimidyl) glutarate (DSG), Disuccinimidyl Tartarate (DST), Dimethyladipimide (DMA), and the like;
2) A sulfydryl-saccharide cross-linking agent: a cross-linking agent based on maleimide and a hydrazide reactive group , which is suitable for connecting sulfydryl with saccharide, for example, N-(β-Maleimidopropionic acid)hydrazide (BMPH), 4-(4-N-maleimidophenyl)-butyric acid hydrazide (MPBH), 3-(2-Pyridyldithio)propionylhydrazide(PDPH) and the like;
3) A sulfydryl-sulfydryl cross-linking agent: a homobifunctional cross-linking agent based on maleimide or a pyridine disulfydryl reactive group, which is suitable for undergoing a specific covalent linkage with the mercaptan (reduced cysteine) of protein or polypeptide to form a stable thioether bond, for example, bis(maleimido)ethane (BMOE), 1,4-Bis(maleimido)butane (BMB), bis-maleimidohexane (BMH), and the like;
4) An amino-sulfydryl cross-linking agent: a heterobifunctional protein cross-linking agent suitable for linking a protein with the primary amine (lysine) and the sulfydryl (cysteine) of another molecule, for example, Succinimidyl iodoacetate (SIA), succinimidyl 3-(2-pyridyldithio)propionate (SPDP), N-α-Maleimidoacetoxy succinimide ester (AMAS), and the like.
5) A carboxyl-amino cross-linking agent: a carbodiimide cross-linking agent , for example, N-Hydroxysuccinimide(NHS), 1-Ethyl-3-(3-dimethylaminopropyl)carbodiimide hydrochloride (EDC), and the like, which is suitable for linking carboxyl (glutamic acid, aspartic acid, C- terminal) with primary amine (lysine, N- terminal);
6) A photoreactive cross-linking agent: a heterobifunctional cross-linking agent for aromatic azide, dimethylenimine and other photoreactions (photoactivations), for example, succinimidyl 4,4'-azipentanoate (SDA), N-5-azido-2-nitrobenzoyloxysuccinimide (ANB-NOS), and the like;
7) A chemoselectivity cross-linking agent : for example, N-azidoacetylmannosamine-tetraacylated (ManNAz), N-azidoacetylgalactosamine tetraacylated (GalNAz), and the like; and
8) A bifunctional cross-linking agent: for example, the two aldehyde groups of glutaraldehyde respectively form schiff bonds with the amino groups on the antigen and a protein.

Preferably, the molar ratio of the marker protein to the antigen is 1:1 -3:1 when the marker protein and the antigen undergo the coupling reaction.

The buffer solution used for the coupling reaction includes, but is not limited to: a PBS buffer solution whose pH is 4.0-6.5, a carbonate buffer solution whose pH is 8.0-9.8, a glutaraldehyde solution whose concentration is 0.5%-5% and pH is 7.0-8.0, and an MES buffer solution whose pH is 4.0-6.0.

According to a typical embodiment of the present disclosure, the labeled-complex intermediate or labeled complex can be placed in a stock solution for storage after it is prepared, and the stock solution including, but not limited to: 0.2-0.5g/L potassium dihydrogen phosphate, 3.0-6.0g/L disodium hydrogen phosphate, 4.0-8.0g/L sodium chloride, 0.5-1.5g/L BSA, 0.25-0.5g/L sodium azide and 0.25-0.5mL/L Tween-20.

According to a typical embodiment of the present disclosure, a detection kit is provided. The kit includes any one of the labeled complexes disclosed herein or a labeled complex prepared using any one of the labeled complex methods disclosed herein. The detection kit may be an Enzyme-linked immunosorbent assay kit, an immune colloidal gold kit, a radioimmunoassay kit or a chemiluminescence immunoassay kit, and of course, the detection kit may be another detection kit which can use the labeled complex disclosed herein to improve the stability and performance of this type of products.

According to a typical embodiment of the present disclosure, the detection kit further includes: a magnetic particle solution coated with an antigen; or a magnetic particle solution coated with avidin and a biotinylated antigen solution; or a magnetic particle solution coated with an anti-fluorescein isothiocyanate antibody and an antigen solution labeled with fluorescein isothiocyanate. For example, if the detection kit is a kit for detecting an HIV-1 antibody, then the detection kit contains the labeled complex of a recombinant HIV-1 antigen. Preferably, the kit further includes a kit buffer solution.

Preferably, the magnetic particle adopted herein is the complex of nano-sized Fe₂O₃ or Fe₃O₄ magnetic nanoparticles and an organic polymer material and has a particle diameter of 0.1-5µm, moreover, the surface of the magnetic particle is modified to carry one or more active groups. The active group includes at least one of carboxyl, hydroxyl and amino. The magnetic particle is a micro-sized solid phase particle with superparamagnetism and a super large amount of protein adsorption capacity. They have the property of rapidly magnetizing under an applied magnetic field and zero remanence after the magnetic field is removed. In the use of the magnetic particle, the surface of the magnetic particle can be linked with an active group through surface modification, thus reducing nonspecific adsorption as well as improving the stability of a system without agglomeration and significantly increasing the bonding rate.

According to a typical embodiment of the present disclosure, the detection kit is a chemiluminiscence immunodiagnosis kit which specifically includes: a magnetic particle solution coated with a recombinant HIV-1 antigen and the labeled complex solution of the recombinant HIV-1 antigen; or a magnetic particle solution coated with avidin, the labeled complex solution of a recombinant HIV-1 antigen and a biotinylated recombinant HIV-1 antigen solution; or a magnetic particle solution coated with an anti-fluorescein isothiocyanate antibody, a recombinant HIV-1 antigen solution labeled with fluorescein isothiocyanate and the labeled complex solution of a recombinant HIV-1 antigen. Preferably, the kit further includes a kit buffer solution.

According to a typical embodiment of the present disclosure, a concentration of the recombinant HIV-1 antigen in a magnetic particle solution coated with the recombinant HIV-1 antigen, that of the avidin in a magnetic particle solution coated with avidin, or that of the anti-fluorescein isothiocyanate antibody in a magnetic particle solution coated with the anti-fluorescein isothiocyanate antibody is 10-200µg/L; a concentration of the magnetic particles is 0.25-1.25mg/mL; and the magnetic particle solution coated with the recombinant HIV-1 antigen, the magnetic particle solution coated with avidin or the magnetic particle solution coated with an anti-fluorescein isothiocyanate antibody contains 0.1-0.3M phosphate buffer solution, 0.3-0.6M NaCl, 0.05%-0.15% of BSA and 0.05-0.10% of TritonX-100, and has a pH value of 7.2-7.8.

According to a typical embodiment of the present disclosure, a concentration of the recombinant HIV-1 antigen in the labeled complex solution of the recombinant HIV-1 antigen is 10-200ug/L, that of the labeled complex of the recombinant HIV-1 antigen is 0.1-1mg/L, the labeled complex diluent of the recombinant HIV-1 antigen contains 0.2-0.6g/L casein, 1.0-3.0g/L Tris-base, 5.0-8.0g/L sodium chloride, 5.0-10.0g/L glycerol, 0.5-0.8mL/L Tween-20, 200-300mM EDTA, 1%-3% of saccharose, 0.2-0.5M glycine and 20-50% of calf serum, and has a pH value of 7.5-8.0.

According to a typical embodiment of the present disclosure, the kit buffer solution contains 1.0-3.0g/L Tris, 0.8-1.6g/L NaCl, 0.05%-0.15% of TritonX-100, 0.5-1.5g/L BSA and 0.05%-0.15% of HCl, and has a pH value of 7.2-7.8, wherein the purity of Tris is equal to or greater than 99%, NaCl is analytically pure, the purity of BSA is equal to or greater than 99%, and HCl is analytically pure and has a concentration of 36wt%-38wt%.

According to a typical embodiment of the present disclosure, in a biotinylated recombinant HIV-1 antigen solution, a concentration of a recombinant HIV-1 antigen is 10-200ug/L, and that of biotin is 0.1-1mg/L; or in a recombinant HIV-1 antigen solution labeled with fluorescein isothiocyanate, a concentration of a recombinant HIV-1 antigen is 10-200ug/L, and that of fluorescein isothiocyanate is 0.1-1mg/L.

According to a typical embodiment of the present disclosure, a magnetic particle solution coated with avidin refers to a magnetic particle solution coated with streptavidin.

According to a typical embodiment of the present disclosure, a reagent or kit (chemiluminescence immunoassay) for detecting an HIV-1 antibody is provided which includes the foregoing recombinant HIV-1 antigen labeled complex. The kit includes the following components:
1) A magnetic particle solution coated with a recombinant HIV-1 antigen
   The concentration of the recombinant HIV-1 antigen: 10-200ug/L
   The concentration of magnetic particles: 0.25-1.25mg/mL
   The magnetic particle solution contains 0.1-0.3M phosphate buffer solution, 0.3-0.6M NaCl, 0.05%-0.15% of BSA and 0.05-0.10% of TritonX-100, and has a pH value of 7.2-7.8.
2) A labeled complex solution of the recombinant HIV-1 antigen
   The concentration of the recombinant HIV-1 antigen: 10-200ug/L
   The concentration of a labeled complex: 0.1-1mg/L
   A labeled complex diluent contains 0.2-0.6g/L casein, 1.0-3.0g/L Tris-base, 5.0-8.0g/L sodium chloride, 5.0-10.0g/L glycerol, 0.5-0.8mL/L Tween-20, 200-300mM EDTA, 1%-3% of saccharose, 0.2-0.5M glycine and 20-50% of calf serum, and has a pH value of 7.5-8.0.
3) a kit buffer solution
   the kit buffer solution contains 1.0-3.0g/L Tris (purity: ≥99%), 0.8-1.6g/L NaCl (analytical pure), 0.05%-0.15% of TritonX-100 and 0.5-1.5g/L BSA (purity: ≥99%) and 0.05%-0.15% of HCl (analytical pure, concentration: 36wt%-38wt%), and has a pH of 7.2-7.8.

The components all contain a preservative which is one of potassium sorbate, sodium benzoate, sodium azide, sodium nitrite and Procilin series, or a mixture thereof.

According to a typical embodiment of the present disclosure, a reagent or kit (chemiluminescence immunoassay) for detecting an HIV-1 antibody is provided which includes the foregoing recombinant HIV-1 antigen labeled complex. The kit includes the following components:
1) a magnetic particle solution coated with streptavidin (SA)
   The concentration of streptavidin: 10-200ug/L
   The concentration of magnetic particles: 0.25-1.25mg/mL
   The magnetic particle solution contains 0.1-0.3M phosphate buffer solution, 0.3-0.6M NaCl, 0.05%-0.15% of BSA and 0.05-0.10% of TritonX-100, and has a pH value of 7.2-7.8.
2) A labeled complex solution of a recombinant HIV-1 antigen
   The concentration of the recombinant HIV-1 antigen: 10-200ug/L
   The concentration of the labeled complex: 0.1-1mg/L
   A labeled complex diluent contains 0.2-0.6g/L casein, 1.0-3.0g/L Tris-base, 5.0-8.0g/L sodium chloride, 5.0-10.0g/L glycerol, 0.5-0.8mL/L Tween-20, 200-300mM EDTA, 1%-3% of saccharose, 0.2-0.5M glycine, 20-50% of calf serum, and has a pH value of 7.5-8.0.
3) a biotinylated recombinant HIV-1 antigen solution
   The concentration of the recombinant HIV-1 antigen: 10-200ug/L
   The concentration of biotin: 0.1-1mg/L

A diluted buffer solution contains 1.0-3.0g/L Tris (purity: ≥99%), 0.8-1.6g/L NaCl (analytical pure), 0.05%-0.15% of TritonX-100 and 0.5-1.5g/L BSA (purity: ≥99%) and 0.05%-0.15% of HCl (analytical pure, concentration:36wt%-38wt% 36%-38%), and has a pH of 7.2-7.8.

The components all contain a preservative which is one of potassium sorbate, sodium benzoate, sodium azide, sodium nitrite and Procilin series, or a mixture thereof.

According to a typical embodiment of the present disclosure, a reagent or kit (chemiluminescence immunoassay) for detecting an HIV-1 antibody is provided which includes the foregoing recombinant HIV-1 antigen labeled complex. The kit includes the following components:
1) A magnetic particle solution coated with an goat anti-FITC
   The concentration of the goat anti-FITC: 10-200ug/L
   The concentration of magnetic particles: 0.25-1.25mg/mL
   The magnetic particle solution contains 0.1-0.3M phosphate buffer solution, 0.3-0.6M NaCl, 0.05%-0.15% of BSA and 0.05-0.10% of TritonX-100 and has a pH value of 7.2-7.8.
2) A labeled complex solution of a recombinant HIV-1 antigen
   The concentration of the recombinant HIV-1 antigen: 10-200ug/L
   The concentration of the labeled complex: 0.1-1mg/L
   The diluent of the labeled complex contains 0.2-0.6g/L casein, 1.0-3.0g/L Tris-base, 5.0-8.0g/L sodium chloride, 5.0-10.0g/L glycerol, 0.5-0.8mL/L Tween-20, 200-300mM EDTA, 1%-3% of saccharose, 0.2-0.5M glycine and 20%-50% of calf serum, and has a pH value of 7.5-8.0.
3) A FITC-labeled recombinant HIV-1 antigen solution
   The concentration of the recombinant HIV-1 antigen: 10-200ug/L
   The concentration of FITC: 0.1-1mg/L

A diluted buffer solution contains 1.0-3.0g/L Tris (purity: ≥99%), 0.8-1.6g/L NaCl (analytical pure), 0.05%-0.15% of TritonX-100 and 0.5-1.5g/L BSA (purity:≥99%) and 0.05%-0.15% of HCl (analytical pure, concentration: 36wt%-38wt%), and has a pH of 7.2-7.8.

The components all contain a preservative which is one of potassium sorbate, sodium benzoate, sodium azide, sodium nitrite and Procilin series, or a mixture thereof.

In the present disclosure, when the antigen is a recombinant HIV-1 antigen, a recombinant HIV-2 antigen, a hepatitis A virus antigen, a hepatitis B virus antigen, a hepatitis C virus antigen, a hepatitis D virus antigen, a hepatitis E virus antigen, a hepatitis G virus antigen, a human T lymphocyte virus antigen, a treponema pallidum antigen, a helicobacter pylori antigen or a human papilloma virus antigen, by following the inventive concept disclosed herein and by reference to an implementation mode or embodiment in which an antigen refers to a recombinant HIV-1 antigen, those of ordinary skill in the art can adapt the solution or the reaction conditions by conventional techniques to achieve the present disclosure.

The beneficial effects of the present disclosure are further elaborated below with reference to embodiments.
1. Sources of raw materials:
   Goat anti-FITC polyclonal antibody: purchased from Beijing Baihao Biotechnology Co., Ltd.;
   Magnetic particle: produced by New Industries Biomedical Engineering Co. Ltd. (Shenzhen, China);
   FITC: purchased from Shanghai Ji'ning Industrial Co., Ltd.
   ABEI: produced by New Industries Biomedical Engineering Co. Ltd. (Shenzhen, China);
   Biotin and streptavidin: both purchased from Roche;
   HIV-1 antibody calibrator (product ID: B65875G): sourced from Meridian;
   Recombinant HIV-1 antigen for coating (product ID: VTI310): sourced from: Meridian;
   Recombinant HIV-1 antigen for being labeled (product ID: R18550): sourced from Meridian.
2. Specific explanations of the sources of a part of raw materials

The recombinant HIV-1 antigen for coating (product ID: VTI310), which is expressed by pichia pastoris, is the recombinant transmembrane protein gp41 of HIV-1, a stock solution contains 1M urea, 0.5M sodium chloride, 0.2M sodium phosphate and has a pH of 7.4±0.2. The recombinant HIV-1 antigen for beinglabeled (product ID: R18550), which is expressed by escherichia coli, is the fused expression of most of the recombinant transmembrane protein gp41 of HIV-1 and the C-terminal of gp120, and the molecular weight is 27.3kDa, and a stock solution contains 50mM Tris, 0.1% of SDS, 5mM DTT and 2.5mM EDTA, and has a pH of 8.0. The HIV-1 antibody calibrator (product ID: B65875G) is a detection sample for sensitivity and stability assessment.

### Embodiment 1

1) 5mg of HRP (M=44000) is weighed and dissolved in 1mL of distilled water (5mg/mL).
2) 0.5mL of newly prepared 0.06M NaIO₄ (M=213.89) solution is added into and uniformly mixed with the foregoing solution, and the obtained mixture is placed still in a dark place for 30 minutes at a temperature of 4°C.
3) 0.5mL of 0.16M ethylene glycol is added (excessive NaIO₄ is removed) into and uniformly mixed with the obtained mixture, and then the mixture is placed still for 30 minutes at room temperature.
4) 1mg of a recombinant HIV-1 antigen (2mg/mL) is dialyzed for 2h at 4°C with 0.05M pH 9.5 carbonate buffer solution, then 1mg of NaIO₄-oxidized HRP is added immediately, and the obtained solution is lightly stirred in a dark place for 2h at room temperature.
5) 50ul of newly prepared 2mg/mL NaBH₄ (M=37.83) solution is added into and uniformly mixed with the obtained solution, and then the mixture is placed still for 2h at 4°C.
6) The obtained solution is loaded into a dialysis bag and dialyzed with 0.01M pH 7.4 PBS overnight at 4°C.
7) A dialysis bag having a molecular weight cutoff (MWCO) of 3500Dlt and a suitable size is selected, wetted, and then tightened at one end, and whether or not the dialysis bag leaks is tested for three times with purified water ( no liquid leakage is appropriate);
8) 0.5mg of the recombinant HIV-1 antigen primarily labeled with HRP is taken, the volume of the recombinant HIV-1 antigen is adjusted to 0.5mL with 0.1mol/L pH 9.5 carbonate buffer solution, the other end of the dialysis bag is tightened, and then the dialysis bag is placed into a pH 9.5 carbonate buffer solution dialysate and dialyzed for 1h at a speed of 400r/min.
9) The dialyzed labeled-complex intermediate is placed into a 2mL of glass bottle, 25ug of ABEI active ester is added into the glass bottle, and then the glass bottle is vibrated at room temperature for the substances therein to react for 1.5h.
10) A purification treatment is performed using a G25 gel column to remove unbound ABEI active ester, then a secondarily labeled HIV labeled complex is obtained.

The foregoing labeled complex is diluted in a buffer solution in a ratio of 1: 400, and then assessed with a Maglumi 2000 Plus automatic analyzer from New Industries Biomedical Engineering Co. Ltd. (Shenzhen, China) based on the HIV-1 antibody detection kit described below. 7 parts of labeled complex solution are simultaneously prepared, and then placed in a 37°C constant temperature and humidity box for an accelerated stability test.

The components of the kit mainly include:
a. A magnetic particle solution coated with a recombinant HIV-1 antigen
   The concentration of the recombinant HIV-1 antigen: 10ug/L
   The concentration of the magnetic particle: 1.0mg/mL
   The magnetic particle solution contains 0.25M phosphate buffer solution, 0.3M NaCl, 0.15% of BSA and 0.05% of TritonX-100, and has a pH value of 7.4.
b. The labeled complex solution with the recombinant HIV-1 antigen secondarily labeled
   The concentration of the recombinant HIV-1 antigen: 50ug/L
   The concentration of the labeled complex: 0.2mg/L
   The diluent of the labeled complex contains 0.2g/L casein, 1.5g/L Tris-base, 6.0 g/L sodium chloride, 10.0g/L glycerol, 0.6mL/L Tween-20, 250mM EDTA, 2% of saccharose, 0.4M glycine and 25% calf serum, and has a pH value of 7.8.
c. A kit buffer solution
   The buffer solution contains 2.0g/L Tris (purity: ≥99%), 1.2g/L NaCl (analytical pure), 0.1 % of TritonX-100 and 0.5g/L BSA (purity: ≥99%) and 0.1 % of HCl (analytical pure, concentration: 36wt%-38wt%), and has a pH of 7.5.
   The components all contain 0.2% of ProClin 300 as a preservative.

### Embodiment 2

1) 5mg of HRP (M=44000) is weighed and dissolved in 1mL of distilled water (5mg/mL).
2) 1.0mL of newly prepared 0.06 M NaIO₄ (M=213.89) solution is added into and uniformly mixed with the foregoing solution, and the obtained mixture is placed still in a dark place for 30 minutes at a temperature of 4°C.
3) 1.0mL of 0.16M ethylene glycol is added into and mixed uniformly with the obtained mixture, and then the mixture is placed still for 30 minutes at room temperature.
4) 1mg of a recombinant HIV-1 antigen (2mg/mL) is dialyzed for 2h at 4°C with 0.05M pH 9.5 carbonate buffer solution, 2mg of NaIO₄-oxidized HRP is added immediately, and then the obtained solution is lightly stirred in a dark place for 2h at room temperature.
5) 100ul of newly prepared 2mg/mL NaBH₄ (M=37.83) solution is added into and uniformly mixed with the obtained solution, and then the mixture is placed still for 2h at 4°C.
6) The obtained solution is loaded into a dialysis bag and dialyzed with 0.01M pH 7.4 PBS overnight at 4°C.
7) A dialysis bag having a MWCO of 3500Dlt and a suitable size is selected, wetted, and then tightened at one end, and whether or not the dialysis bag leaks is tested for three times with purified water (no liquid leakage is appropriate);
8) 0.5mg of the recombinant HIV-1 antigen primarily labeled with HRP is taken, and the volume of the recombinant HIV-1 antigen is adjusted to 0.5mL with 0.1mol/L pH 9.5 carbonate buffer solution, the other end of the dialysis bag is tightened, and then the dialysis bag is placed into a pH 9.5 carbonate buffer solution dialysate and dialyzed for 1h at a speed of 400r/min.
9) The dialyzed labeled-complex intermediate is placed into a 2mL of glass bottle, 25ug of ABEI active ester is added into the glass bottle, and then the glass bottle is vibrated at room temperature for the substances therein to react for 1.5h.
10) A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

### Embodiment 3

1) 5mg of HRP (M=44000) is weighed and dissolved in 1mL of distilled water (5mg/mL).
2) 1.5mL of newly prepared 0.06 M NaIO₄ (M=213.89) solution is added into and uniformly mixed with the foregoing solution, and the obtained mixture is placed still in a dark place for 30 minutes at a temperature of 4°C.
3) 1.5mL of 0.16M ethylene glycol is added into and mixed uniformly with the obtained mixture, and then the mixture is placed still for 30 minutes at room temperature.
4) 1mg of a recombinant HIV-1 antigen (2mg/mL) is dialyzed for 2h at 4°C with 0.05M pH 9.5 carbonate buffer solution, then 3mg of NaIO₄-oxidized HRP is added immediately, and the obtained solution is lightly stirred in a dark place for 2h at room temperature.
5) 150ul of newly prepared 2mg/mL NaBH₄ (M=37.83) solution is added into and uniformly mixed with the obtained solution, and then the mixture is placed still for 2h at 4°C.
6) The obtained solution is loaded into a dialysis bag and dialyzed with 0.01M pH 7.4 PBS overnight at 4°C.
7) A dialysis bag having a MWCO of 3500Dlt and a suitable size is selected, wetted, and then tightened at one end, and whether or not the dialysis bag leaks is tested for three times with purified water (no liquid leakage is appropriate).
8) 0.5mg of the recombinant HIV-1 antigen primarily labeled with HRP is taken, and the volume of the recombinant HIV-1 antigen is adjusted to 0.5mL with 0.1mol/L pH 9.5 carbonate buffer solution, the other end of the dialysis bag is tightened, and then the dialysis bag is placed into a pH 9.5 carbonate buffer solution dialysate and dialyzed for 1h at a speed of 400r/min.
9) The dialyzed labeled-complex intermediate is placed into a 2mL of glass bottle, 25ug of ABEI active ester is added into the glass bottle, and then the glass bottle is vibrated at room temperature for the substances therein to react for 1.5h.
10) A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

### Embodiment 4

1) 5mg of HRP (M=44000) is weighed and dissolved in 1mL of distilled water (5mg/mL).
2) 1.0mL of newly prepared 0.06M NaIO₄ (M=213.89) solution is added into and uniformly mixed with the foregoing solution, and the obtained mixture is placed still in a dark place for 30 minutes at a temperature of 4°C.
3) 1.5mL of 0.16M ethylene glycol is added into and mixed uniformly with the obtained mixture, and then the mixture is placed still for 30 minutes at room temperature.
4) 1mg of a recombinant HIV-1 antigen (2mg/mL) is dialyzed for 2h at 4°C with 0.05M pH 9.5 carbonate buffer solution, and 2mg of NaIO₄-oxidized HRP is added immediately, and then the obtained solution is lightly stirred in a dark place for 2h at room temperature.
5) 100ul of newly prepared 2mg/mL NaBH₄ (M=37.83) is added into and uniformly mixed with the obtained solution, and then the mixture is placed still for 2h at 4°C.
6) The obtained solution is loaded into a dialysis bag and dialyzed with 0.01M pH 7.4 PBS overnight at 4°C.
7) A dialysis bag having a MWCO of 3500Dlt and a suitable size is selected, wetted, and then tightened at one end, and whether or not the dialysis bag leaks is tested for three times using purified water (it is preferred that the dialysis bag leaks no liquid).
8) 0.5mg of the recombinant HIV-1 antigen primarily labeled with HRP is taken, the volume of the recombinant HIV-1 antigen is increased to 0.5mL with the use of 0.1mol/L carbonate buffer solution whose pH is 9.5, the other end of the dialysis bag is tightened, and then the dialysis bag is placed into a carbonate buffer solution dialyzate whose pH is 9.5 to dialyze for 1h at a speed of 400r/min.
9) The dialyzed labeled-complex intermediate is placed into a 2mL of glass bottle, 25ug of ABEI active ester is added into the glass bottle, and then the glass bottle is vibrated at room temperature for the substances therein to react for 1.5h.
10) A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

The components of the kit mainly include:
a. A magnetic particle solution coated with streptavidin (SA)
   The concentration of the streptavidin: 20ug/L
   The concentration of the magnetic particle: 1.0mg/mL
   The magnetic particle solution contains 0.25M phosphate buffer solution, 0.3M NaCl, 0.15% of BSA and 0.05% of TritonX-100, and has a pH value of 7.4.
b. The labeled complex solution with the recombinant HIV-1 antigen secondarily labeled
   The concentration of the recombinant HIV-1 antigen: 10ug/L
   The concentration of the labeled complex: 0.2mg/L
   The diluent of the labeled complex contains 0.2g/L casein, 1.5g/L Tris-base, 6.0 g/L sodium chloride, 10.0g/L glycerol, 0.6mL/L Tween-20, 250mM EDTA, 2% of saccharose, 0.4M glycine and 25% calf serum, and has a pH value of 7.8.
c. A biotinylated recombinant HIV-1 antigen solution
   The concentration of the recombinant HIV-1 antigen: 10ug/L
   The concentration of the biotin: 0.1ug/L
   The diluent of the biotinylated recombinant HIV-1 antigen solution contains 2.0g/L Tris (purity: ≥99%), 1.2g/L NaCl (analytical pure), 0.1% of TritonX-100 and 0.5g/L BSA (purity: ≥99%) and 0.1% of HCl (analytical pure, concentration: 36wt%-38wt%), and has a pH of 7.5.
   The components all contain 0.2% of ProClin 300 as a preservative.

### Embodiment 5

1) 5mg of HRP (M=44000) is weighed and dissolved in 1mL of distilled water (5mg/mL).
2) 1.0mL of newly prepared 0.06 M NaIO₄ (M=213.89) solution is added into and uniformly mixed with the foregoing solution, and the obtained mixture is placed still in a dark place for 30 minutes at a temperature of 4°C.
3) 1.5mL of 0.16M ethylene glycol is added into and mixed uniformly with the obtained mixture, and then the mixture is placed still for 30 minutes at room temperature.
4) 1mg of a recombinant HIV-1 antigen (2mg/mL) is dialyzed for 2h at 4°C with the use of 0.05M carbonate buffer solution whose pH is 9.5, and 2mg of NaIO₄-oxidized HRP is added immediately, and then the obtained solution is lightly stirred in a dark place for 2h at room temperature.
5) 100ul of newly prepared 2mg/mL NaBH₄ (M=37.83) is added into and uniformly mixed with the obtained solution, and then the mixture is placed still for 2h at 4°C.
6) The obtained solution is loaded into a dialysis bag and dialyzed with 0.01M pH 7.4 PBS overnight at 4°C.
7) A dialysis bag having a MWCO of 3500Dlt and a suitable size is selected, wetted, and then tightened at one end, and whether or not the dialysis bag leaks is tested for three times with purified water (no liquid leakage is appropriate);
8) 0.5mg of the recombinant HIV-1 antigen primarily labeled with HRP is taken, the volume of the recombinant HIV-1 antigen is adjusted to 0.5mL with 0.1mol/L pH 9.5 carbonate buffer solution, the other end of the dialysis bag is tightened, and then the dialysis bag is placed into a pH 9.5 carbonate buffer solution dialysate and dialyzed for 1h at a speed of 400r/min.
9) The dialyzed labeled-complex intermediate is placed into a 2mL of glass bottle, 25ug of ABEI active ester is added into the glass bottle, and then the glass bottle is vibrated at room temperature for the substances therein to react for 1.5h.
10) A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

The components of the kit mainly include:
a. A magnetic particle solution coated with an goat anti-FITC
   The concentration of the goat anti-FITC: 20ug/L
   The concentration of magnetic particles: 1.0mg/mL
   The magnetic particle solution contains 0.25M phosphate buffer solution, 0.3M NaCl, 0.15% of BSA and 0.05% of TritonX100, and has a pH value of 7.4.
b. The labeled complex solution with the recombinant HIV-1 antigen secondarily labeled
   The concentration of the recombinant HIV-1 antigen: 10ug/L
   The concentration of the labeled complex: 0.2mg/L
   The diluent of the labeled complex contains 0.2g/L casein, 1.5g/L Tris-base, 6.0g/L sodium chloride, 10.0g/L glycerol, 0.6mL/L Tween-20, 250mM EDTA, 2% of saccharose, 0.4M glycine and 25% calf serum, and has a pH value of 7.8.
c. An FITC-labeled recombinant HIV-1 antigen solution
   The concentration of the recombinant HIV-1 antigen: 10ug/L
   The concentration of FITC: 0.1ug/L
d. A kit buffer solution
   The buffer solution contains 2.0g/L Tris (purity: ≥99%), 1.2g/L NaCl (analytical pure), 0.1% of TritonX-100 and 0.5g/L BSA (purity:≥99%) and 0.1% of HCl (analytical pure, concentration: 36wt%-38wt%), and has a pH of 7.5.
   The components all contain 0.2% of ProClin 300 as a preservative.

### Embodiment 6

The labeling of HRP in embodiment 1 is replaced with the labeling of alkaline phosphatase, and the labeled-complex intermediate is secondarily labeled with ABEI. Specific labeling steps are as follows:
100µL of alkaline phosphatase is added into 300µL of 10mM PBS (pH: 7.2), then 40µL of 25% glutaraldehyde is added, they are uniformly mixed and reacted for 20h at 4°C, the obtained solution is dialyzed into 10mM PBS (pH: 7.2), and the dialysate solution is changed for four times within 24h; a recombinant HIV-1 antigen is prepared to have a concentration of 2mg/mL with 10mM PBS (pH: 7.2), 250µL of the recombinant HIV-1 antigen is added into and uniformly mixed with the dialyzed alkaline phosphatase (final concentration of the recombinant HIV-1 antigen is 5000 U AP/mg), the mixture is reacted for 24h at 4°C. The dialyzed labeled-complex intermediate is placed into a 2mL of glass bottle, 25µg ABEI active ester is added into the glass bottle, and then the glass bottle is vibrated at room temperature for the substances therein to react for 1.5h. A purification treatment is performed with a G25 gel column to remove unbound ABEI active ester.

The foregoing labeled complex is prepared into a kit according to the scheme provided in embodiment 1, and the sensitivity and the accelerated thermal stability of the kit are assessed.

### Embodiment 7 (not part of the invention)

The labeling of HRP in embodiment 1 is replaced with the labeling of BSA, and the labeled-complex intermediate is secondarily labeled with ABEI. Specific labeling steps are as follows: 100µL of BSA is added into 300µL of 10mM PBS (pH: 7.2), then 40µL of 25% glutaraldehyde is added, they are uniformly mixed and reacted for 20h at 4 °C, the obtained solution is dialyzed into 10mM PBS (pH: 7.2), and the dialysate solution is changed for four times within 24h; a recombinant HIV-1 antigen is prepared to have a concentration of 2mg/mL with 10mM PBS (pH: 7.2), 250µL of the recombinant HIV-1 antigen is added into and uniformly mixed with the dialyzed BSA (final concentration is 1mg BSA/mg recombinant HIV-1 antigen), the mixture is reacted for 24h at 4°C. The dialyzed labeled-complex intermediate is placed into a 2mL of glass bottle, 25µg ABEI active ester is added into the glass bottle, and then the glass bottle is vibrated at room temperature for the substances therein to react for 1.5h. A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

### Comparative example 1

1) A dialysis bag having a MWCO of 3500Dlt and a suitable size is selected, wetted, and then tightened at one end, and whether or not the dialysis bag leaks is tested for three times using purified water (no liquid leakage is appropriate);
2) 0.5mg of a recombinant HIV-1 antigen (2mg/mL) is taken, the volume of the recombinant HIV-1 antigen is adjusted to 0.5mL with 0.1mol/L pH 9.5 carbonate buffer solution, the other end of the dialysis bag is tightened, and then the dialysis bag is placed into a pH 9.5 carbonate buffer solution dialysate and dialyzed for 1h at a speed of 400r/min.
3) The dialyzed labeled-complex intermediate is placed into a 2mL of glass bottle, 25ug of ABEI active ester is added into the glass bottle, and then the glass bottle is vibrated at room temperature for the substances therein to react for 1.5h.
4) A purification treatment is performed using a G25 gel column to remove unbound ABEI active ester.

The foregoing labeled complex is prepared into a kit according to the scheme provided in embodiment 1, and the sensitivity and the accelerated thermal stability of the kit are assessed.

### Comparative example 2

The labeled complex which is directly labeled in the foregoing comparative example 1 is prepared into a kit according to the scheme provided in embodiment 4, and the sensitivity and the accelerated thermal stability of the kit are assessed.

### Comparative example 3

The labeled complex which is directly labeled in the foregoing comparative example 1 is prepared into a kit according to the scheme provided in embodiment 5, and the sensitivity and the accelerated thermal stability of the kit are assessed.

### Experimental data and result analysis

A medium positive sample S1 is diluted with an HIV negative sample S6 to obtain S2, and by sequentially diluted, S3, S4 and S5 are obtained. These samples are used as detection samples, and their relative light unit (RLU) are measured with a Maglumi 2000 Plus automatic analyzer from New Industries Biomedical Engineering Co. Ltd. (Shenzhen, China).

Results of detection on sensitivities of the products obtained in the foregoing embodiments and comparative examples are shown in Table 1 to Table 3.

**Table 1**

| Sensitivity reference | Comparative example 1 | Embodiment 1 | | Embodiment 2 | | Embodiment 3 | |
|---|---|---|---|---|---|---|---|
| | (RLU) | (RLU) | Relative deviation | (RLU) | Relative deviation | (RLU) | Relative deviation |
| S1 | 215032 | 338917 | 58% | 363668 | 69% | 330832 | 54% |
| S2 | 126390 | 171963 | 36% | 194806 | 54% | 169737 | 34% |
| S3 | 70134 | 98012 | 40% | 114540 | 63% | 95831 | 37% |
| S4 | 38648 | 56675 | 47% | 64515 | 67% | 55820 | 44% |
| S5 | 23580 | 35466 | 50% | 37336 | 58% | 34896 | 48% |
| S6 | 11845 | 12135 | 2% | 12657 | 7% | 12302 | 4% |

**Table 2**

| Sensitivity reference | Comparative example 2 | Embodiment 4 | | Comparative example 3 | Embodiment 5 | |
|---|---|---|---|---|---|---|
| | (RLU) | (RLU) | Relative deviation | (RLU) | (RLU) | Relative deviation |
| S1 | 206578 | 318345 | 54% | 230434 | 343636 | 49% |
| S2 | 126395 | 174804 | 38% | 137746 | 184701 | 34% |
| S3 | 64710 | 89725 | 39% | 64193 | 103566 | 61% |
| S4 | 36257 | 48492 | 34% | 39233 | 54586 | 39% |
| S5 | 21461 | 26130 | 22% | 22650 | 34558 | 53% |
| S6 | 12399 | 11762 | -5% | 12456 | 12055 | -3% |

**Table 3**

| Sensitivity reference | Comparative example 1 | Embodiment 6 | | Embodiment 7 | |
|---|---|---|---|---|---|
| | (RLU) | (RLU) | Relative deviation | (RLU) | Relative deviation |
| S1 | 215032 | 315787 | 47% | 328770 | 53% |
| S2 | 126390 | 169209 | 34% | 177332 | 40% |
| S3 | 70134 | 94173 | 34% | 102532 | 46% |
| S4 | 38648 | 57626 | 49% | 54657 | 41% |
| S5 | 23580 | 31023 | 32% | 33609 | 43% |
| S6 | 11845 | 12036 | 2% | 11785 | -1% |

From the results in Table 1, it can be seen that the feed ratio of the marker protein HRP in embodiment 1, embodiment 2 and embodiment 3 shows it is better when the feed ratio of HIV-1 recombinant antigen and HRP is 1: 2 than when the feed ratio is 1:1 and 1:3; at the same time, these three embodiments have a more substantial improvement than comparative example 1.

The results in Table 2 show that the results of embodiment 4 and comparative example 2 and the results of embodiment 5 and comparative example 3 further show that a recombinant HIV-1 antigen with a secondarily labeled labeled complex is much more sensitive in HIV-1 antibody detection.

The results in Table 3 show that in embodiment 6 and in embodiment 7, different marker protein ALP and BSA are selected for a primary labeling marker protein and ABEI for the subsequent secondary labeling, compared with the direct labeling of comparative example 1, the detection sensitivity is obviously improved (embodiment 7 is not part of the invention).

Thus, the detection sensitivity by a secondarily labeled labeled complex is higher than that of direct labeling. Because by labeling an antigen with a marker protein, the antigen carries a plurality of marker proteins, each of which is bound with a plurality of signal generation substances during a secondary signal generation substance labeling process to generate an effective signal amplification effect, thus improving the sensitivity of HIV-1 antibody detection and helping to shorten the window period of HIV-1 antibody detection.

The reagent is placed in a 37°C constant temperature and humidity box for a 8-day accelerated test. High positive samples are taken as detection samples, and relative light unit (RLU) presented before and after the accelerated test are measured with a Maglumi 2000 Plus automatic analyzer from New Industries Biomedical Engineering Co. Ltd. (Shenzhen, China).

Tables 4-6 show the comparison of the thermal stabilities presented in high-temperature accelerated tests of different embodiments.

**Table 4**

| Embodiment | Comparative example 1 | | Embodiment 1 | | Embodiment 2 | | Embodiment 3 | |
|---|---|---|---|---|---|---|---|---|
| Treated at 37°C | Detected value (RLU) | Deviation (%) | Detected value (RLU) | Deviation (%) | Detected value (RLU) | Deviation (%) | Detected value (RLU) | Deviation (%) |
| Day 0 | 623002 | - | 1012527 | - | 1132528 | - | 972531 | - |
| Day 1 | 452922 | -37.55% | 1007465 | -0.50% | 1123468 | -0.81% | 957943 | -1.52% |
| Day 2 | 414424 | -50.33% | 998398 | -1.42% | 1114480 | -1.62% | 956027 | -1.73% |
| Day 3 | 389558 | -59.93% | 990410 | -2.23% | 1105564 | -2.44% | 947423 | -2.65% |
| Day 4 | 365016 | -70.68% | 982487 | -3.06% | 1098931 | -3.06% | 940791 | -3.37% |
| Day 5 | 338370 | -84.12% | 962837 | -5.16% | 1086842 | -4.20% | 923857 | -5.27% |
| Day 6 | 308932 | -101.66% | 955135 | -6.01% | 1081408 | -4.73% | 922009 | -5.48% |
| Day 7 | 284217 | -119.20% | 953224 | -6.22% | 1074920 | -5.36% | 921087 | -5.59% |

**Table 5**

| Embodiment 1 | Comparative example 2 | | Embodiment 4 | | Comparative example 3 | | Embodiment 5 | |
|---|---|---|---|---|---|---|---|---|
| Treated at 37°C | Detected value (RLU) | Deviation (%) | Detected value (RLU) | Deviation (%) | Detected value (RLU) | Deviation (%) | Detected value (RLU) | Deviation (%) |
| Day 0 | 621248 | - | 1032362 | | 603357 | - | 1089629 | - |
| Day 1 | 444814 | -39.66% | 1010683 | -2.15% | 432004 | -39.66% | 1076553 | -1.21% |
| Day 2 | 406115 | -52.97% | 999565 | -3.28% | 401331 | -50.34% | 1072247 | -1.62% |
| Day 3 | 378499 | -64.13% | 985571 | -4.75% | 368824 | -63.59% | 1051874 | -3.59% |
| Day 4 | 353140 | -75.92% | 979658 | -5.38% | 345588 | -74.59% | 1050822 | -3.69% |
| Day 5 | 329126 | -88.76% | 974760 | -5.91% | 316904 | -90.39% | 1028755 | -5.92% |
| Day 6 | 301809 | -105.84% | 966961 | -6.76% | 290601 | -107.62% | 1022582 | -6.56% |
| Day 7 | 287925 | -115.77% | 959226 | -7.62% | 274037 | -120.17% | 1017470 | -7.09% |

**Table 6**

| Embodiment 1 | Embodiment 6 | | Embodiment 7 | | Comparative example 1 | |
|---|---|---|---|---|---|---|
| Treated at 37°C | Detected value (RLU) | Deviation (%) | Detected value (RLU) | Deviation (%) | Detected value (RLU) | Deviation (%) |
| Day 0 | 1042528 | - | 1072526 | - | 623002 | - |
| Day 1 | 1037315 | -0.50% | 1041423 | -2.99% | 452922 | -37.55% |
| Day 2 | 1015531 | -2.66% | 1009139 | -6.28% | 414424 | -50.33% |
| Day 3 | 986081 | -5.72% | 1002075 | -7.03% | 389558 | -59.93% |
| Day 4 | 981150 | -6.26% | 992054 | -8.11% | 365016 | -70.68% |
| Day 5 | 970358 | -7.44% | 976181 | -9.87% | 338370 | -84.12% |
| Day 6 | 969387 | -7.54% | 972277 | -10.31% | 308932 | -101.66% |
| Day 7 | 959694 | -8.63% | 963526 | -11.31% | 284217 | -119.20% |

It can be known from Tables 4, 5 and 6 that with a secondarily labeled labeled complex, a recombinant HIV-1 antigen retains high immunoreactivity, decreasing about 10% of immunoreactivity after undergoing a 7-day accelerated test at a high temperature of 37 °C, whereas a directly labeled labeled complex decreases about 40% of immunoreactivity on the first day of the same test and takes on a trend of immunoreactivity diminishing; the comparison results reveal that the stability of a secondarily labeled labeled complex is improved significantly (embodiment 7 is not part of the invention).

When a recombinant HIV-1 antigen is affected by a physical or chemical factor, the secondary, tertiary and quaternary structures within their molecules will change,, then the conformation of the antigen is changed, leading to the antigen epitope being covered and leading to the decrease or loss of immunological reactivity of the antigen. However, by coupling a marker protein with functional groups of the antigen, the conformation of the antigen is maintained, or although the conformation of the antigen is changed, the epitope is not covered, thus keeping the immunoreactivity of the antigen.

It can be seen from the above description that the foregoing embodiments of the present disclosure achieve the following technical effects:
1) The stability of the labeled complex of a recombinant antigen is improved through a secondary labeling, which is beneficial to guarantee the performance of a kit;
2) Detection sensitivity is improved, which helps to shorten the detection window period and save raw materials;
3) The problems of indirect labeling including the necessity of looking for a proper small molecule or a tag protein as well as corresponding recognizable ligand, the raise of the difficulty of recombinant antigen expression and the increase of components of a reagent are avoided marker protein;
4) Being secondarily labeled, the HIV antigen retains highly immunoreactivity, moreover, the stability of the labeled complex is improved obviously;
5) The problems are solved that the stability and sensitivity of the labeled complex of a directly labeled HIV antigen are not high enough to meet the requirements on the performance of a reagent.

The mentioned above is only preferred embodiments of the disclosure but not limitation to the disclosure, it should be appreciated that various modification and variations can be devised by those of ordinary skill in the art. The scope of the present disclosure is defined by the appended claims.

## Claims

1. A labeled complex, comprising:
a labeled-complex intermediate, wherein the labeled-complex intermediate comprises an antigen and a marker protein,, wherein the marker protein has a functional group on the surface thereof, and the functional group is one or more of carboxyl, amino, hydroxyl, sulfydryl, aldehyde group, glycosyl and imidazolyl, and the functional group is covalently-coupled with free amino, carboxyl, sulfydryl, aldehyde group or glycosyl on the antigen, and wherein the marker protein is horse radish peroxidase, alkaline phosphatase, acid phosphatase, glucose oxidase or β-galactosidase; and
a signal generation substance covalently-coupled with the marker protein of the labeled-complex intermediate to form the labeled complex;
wherein the marker protein and the signal generation substance are different substances.

2. The labeled complex according to claim 1, wherein the antigen is a recombinant antigen or a natural antigen;
preferably, the antigen is a recombinant HIV-1 antigen, a recombinant HIV-2 antigen, a hepatitis A virus antigen, a hepatitis B virus antigen, a hepatitis C virus antigen, a hepatitis D virus antigen, a hepatitis E virus antigen, a hepatitis G virus antigen, a human T lymphocyte virus antigen, a treponema pallidum antigen, a helicobacter pylori antigen or a human papilloma virus antigen, and preferably, the antigen is the recombinant HIV-1 antigen or the recombinant HIV-2 antigen.

3. The labeled complex according to any one of claims 1-2, wherein the signal generation substance is luminal, isoluminol, a isoluminol derivate, alkaline phosphatase, horse radish peroxidase, a fluorescent substance, a rare earth ion, a rare earth ion chelate ligand, acridinium ester, a acridinium ester derivate, or tris (bipyridine) ruthenium, and preferably, the isoluminol derivate is N-(4-aminobutyl)-N-ethylisoluminol.

4. A method for preparing the labeled complex of any one of claims 1-3, comprising the following steps of:
performing a covalent-coupling reaction between a marker protein having a functional group on the surface thereof and an antigen to obtain a labeled-complex intermediate; and
performing a covalent-coupling reaction between a signal generation substance and the marker protein of the
labeled-complex intermediate to obtain the labeled complex;
wherein the functional group is one or more of carboxyl, amino, hydroxyl, sulfydryl, aldehyde group, glycosyl and imidazolyl, and the functional group is covalently-coupled with free amino, carboxyl, sulfydryl, aldehyde group or glycosyl on the antigen, and
wherein the marker protein is horse radish peroxidase, alkaline phosphatase, acid phosphatase, glucose oxidase or β-galactosidase;
wherein the marker protein and the signal generation substance are different substances.

5. The method according to claim 4, wherein the method used for the covalent-coupling reaction is a mixed anhydride method, a carbodiimide method, a glutaraldehyde method, a glutaric anhydride method, a diazotization method, a succinic anhydride method, a carbonyl-diimidazole method or a sodium-periodate method,
and preferably, a cross-linking agent used in the covalent-coupling reaction is a homobifunctional amino reactive protein cross-linking agent based on NHS-ester and an imidoate reactive group, a sulfydryl-saccharide cross-linking agent based on maleimide and a hydrazide reactive group, a homobifunctional amino reactive protein cross-linking agent based on maleimide or a pyridine disulfydryl reactive group, a heterobifunctional protein cross-linking agent suitable for linking a protein with a primary amine and a sulfydryl of an another molecule, a carbodiimide cross-linking agent suitable for linking carboxyl with primary amine, a heterobifunctional cross-linking agent included in photoreactive cross-linking agents, a chemoselectivity cross-linking agent or a bifunctional cross-linking agent.

6. The method according to claim 4, wherein a molar ratio of the marker protein to the antigen is 1:1 ~ 3:1 when performing the covalent-coupling reaction between the marker protein and the antigen;
preferably, a buffer solution used in the covalent-coupling reaction is a PBS buffer solution whose pH is 4.0-6.5, a carbonate buffer solution whose pH is 8.0-9.8, a glutaraldehyde solution whose concentration is 0.5%-5% and pH is 7.0-8.0, or an MES buffer solution whose pH is 4.0-6.0.

7. The method according to claim 4, wherein after prepared, the labeled-complex intermediate or the labeled complex is placed in a stock solution for storage, and the stock solution comprises: 0.2-0.5g/L potassium dihydrogen phosphate, 3.0-6.0g/L disodium hydrogen phosphate, 4.0-8.0g/L sodium chloride, 0.5-1.5g/L BSA, 0.25-0.5g/L sodium azide and 0.25-0.5mL/L Tween-20.

8. A detection kit, comprising: the labeled complex of any one of claims 1-3.

9. The detection kit according to claim 8, further comprising:
a magnetic particle solution coated with an antigen; or
a magnetic particle solution coated with streptavidin and a biotinylated antigen solution; or
a magnetic particle solution coated with an anti-fluorescein isothiocyanate antibody and an antigen solution labeled with fluorescein isothiocyanate.

10. The detection kit according to claim 9, wherein the detection kit is a kit for detecting an HIV-1 antibody, comprising the labeled complex of a recombinant HIV-1 antigen;
preferably, the kit specifically comprises:
a magnetic particle solution coated with a recombinant HIV-1 antigen and the labeled complex solution of the recombinant HIV-1 antigen; or
a magnetic particle solution coated with streptavidin, the labeled complex solution of a recombinant HIV-1 antigen and a biotinylated recombinant HIV-1 antigen solution; or
a magnetic particle solution coated with an anti-fluorescein isothiocyanate antibody, a recombinant HIV-1 antigen solution labeled with fluorescein isothiocyanate and the labeled complex solution of the recombinant HIV-1 antigen;
preferably, a concentration of the recombinant HIV-1 antigen in the magnetic particle solution coated with the recombinant HIV-1 antigen is 10-200µg/L;
preferably, a concentration of the magnetic particles is 0.25-1.25mg/mL;
preferably, a concentration of the recombinant HIV-1 antigen in the labeled complex solution of the recombinant HIV-1 antigen is 10-200µg/L;
preferably, a concentration of the labeled complex of the recombinant HIV-1 antigen is 0.1-1mg/L;
preferably, a concentration of the recombinant HIV-1 antigen in the biotinylated recombinant HIV-1 antigen solution is 10-200µg/L, and a concentration of biotin is 0.1-1mg/L; and
preferably, a concentration of the recombinant HIV-1 antigen in the recombinant HIV-1 antigen solution labeled with fluorescein isothiocyanate is 10-200µg/L, and a concentration of the fluorescein isothiocyanate is 0.1-1 mg/L.

11. The application of the kit of any one of claims 8-10 in immunodetection, wherein a marker protein and a detected protein are non-homologous proteins.

12. The application according to claim 11, wherein the kit is used for detecting an HIV-1 antibody, an HIV-2 antibody, a hepatitis A virus, a hepatitis B virus, a hepatitis C virus, a hepatitis D virus, a hepatitis E virus, a hepatitis G virusn, a human T lymphocyte virus, a treponema pallidum antibody, a helicobacter pylori antibody or a human papilloma virus antibody;
preferably, the kit is applied to a semi-automatic or automatic immune analyzer.

13. A detection system, comprising: the kit of any one of claims 8-10 and a semi-automatic or automatic immune analyzer.

## Revendications

1. Complexe marqué, comprenant :
un intermédiaire de complexe marqué, dans lequel l'intermédiaire de complexe marqué comprend un antigène et une protéine marqueur, dans lequel la protéine marqueur a un groupe fonctionnel sur sa surface, et le groupe fonctionnel est un ou plusieurs parmi un groupe carboxyle, amino, hydroxyle, sulfydryle, aldéhyde, glycosyle et imidazolyle, et le groupe fonctionnel est couplé de manière covalente avec un groupe amino, carboxyle, sulfydryle, aldéhyde libre ou glycosyle sur l'antigène, et dans lequel la protéine marqueur est la peroxydase de raifort, la phosphatase alcaline, la phosphatase acide, la glucose oxydase ou la β-galactosidase ; et
une substance génératrice de signal couplée de manière covalente à la protéine marqueur de l'intermédiaire de complexe marqué pour former le complexe marqué ;
dans lequel la protéine marqueur et la substance génératrice de signal sont des substances différentes.

2. Complexe marqué selon la revendication 1, dans lequel l'antigène est un antigène recombinant ou un antigène naturel ;
de préférence, l'antigène est un antigène recombinant du VIH-1, un antigène recombinant du VIH-2, un antigène du virus de l'hépatite A, un antigène du virus de l'hépatite B, un antigène du virus de l'hépatite C, un antigène du virus de l'hépatite D, un antigène du virus de l'hépatite E, un antigène du virus de l'hépatite G, un antigène du virus des lymphocytes T humains, un antigène de Treponema pallidum, un antigène d' Helicobacter pylori ou un antigène du virus du papillome humain, et de préférence, l'antigène est l'antigène recombinant du VIH-1 ou l'antigène recombinant du VIH-2.

3. Complexe marqué selon l'une quelconque des revendications 1 et 2, dans lequel la substance génératrice de signal est le luminal, l'isoluminol, un dérivé d'isoluminol, la phosphatase alcaline, la peroxydase de raifort, une substance fluorescente, un ion de terre rare, un ligand chélate d'ion de terre rare, un ester d'acridinium, un dérivé d'ester d'acridinium ou du tris(bipyridine)ruthénium, et de préférence, le dérivé d'isoluminol est le N-(4-aminobutyl)-N-éthylisoluminol.

4. Procédé de préparation du complexe marqué selon l'une quelconque des revendications 1 à 3, comprenant les étapes suivantes :
l'exécution d'une réaction de couplage covalent entre une protéine marqueur ayant un groupe fonctionnel sur sa surface et un antigène pour obtenir un intermédiaire de complexe marqué ; et
l'exécution d'une réaction de couplage covalent entre une substance génératrice de signal et la protéine marqueur de l'intermédiaire de complexe marqué pour obtenir le complexe marqué ;
dans lequel le groupe fonctionnel est un ou plusieurs parmi un groupe carboxyle, amino, hydroxyle, sulfhydryle, aldéhyde, glycosyle et imidazolyle, et le groupe fonctionnel est couplé de manière covalente avec un groupe amino, carboxyle, sulfhydryle, aldéhyde libre ou glycosyle sur l'antigène, et
dans lequel la protéine marqueur est la peroxydase de raifort, la phosphatase alcaline, la phosphatase acide, la glucose oxydase ou la β-galactosidase ;
dans lequel la protéine marqueur et la substance génératrice de signal sont des substances différentes.

5. Procédé selon la revendication 4, dans lequel le procédé utilisé pour la réaction de couplage covalent est un procédé à l'anhydride mixte, un procédé au carbodiimide, un procédé au glutaraldéhyde, un procédé à l'anhydride glutarique, un procédé de diazotation, un procédé à l'anhydride succinique, un procédé au carbonyl-diimidazole ou un procédé au periodate de sodium,
et de préférence, un agent de réticulation utilisé dans la réaction de couplage covalent est un agent de réticulation de protéine amino réactif homobifonctionnel à base d'ester de NHS et d'un groupe réactif imidoate, un agent de réticulation de sulfhydrile-saccharide à base de maléimide et d'un groupe réactif hydrazide, un agent de réticulation de protéine amino réactif homobifonctionnel à base de maléimide ou d'un groupe réactif pyridine disulfhydryle, un agent de réticulation de protéine hétérobifonctionnel approprié pour lier une protéine à une amine primaire et un sulfhydryle d'une autre molécule, un agent de réticulation carbodiimide approprié pour lier un carboxyle à une amine primaire, un agent de réticulation hétérobifonctionnel inclus dans des agents de réticulation photoréactifs, un agent de réticulation de chimiosélectivité ou un agent de réticulation bifonctionnel.

6. Procédé selon la revendication 4, dans lequel un rapport molaire de la protéine marqueur à l'antigène est de 1:1 à environ 3:1 lors de l'exécution de la réaction de couplage covalent entre la protéine marqueur et l'antigène ;
de préférence, une solution tampon utilisée dans la réaction de couplage covalent est une solution tampon de PBS dont le pH est de 4,0 à 6,5, une solution tampon de carbonate dont le pH est de 8,0 à 9,8, une solution de glutaraldéhyde dont la concentration est de 0,5 % à 5 % et le pH est de 7,0 à 8,0, ou une solution tampon de MES dont le pH est de 4,0 à 6,0.

7. Procédé selon la revendication 4, dans lequel, après préparation, l'intermédiaire de complexe marqué ou le complexe marqué est placé dans une solution mère pour stockage, et la solution mère comprend : 0,2 à 0,5 g/l de dihydrogénophosphate de potassium, 3,0 à 6,0 g/l d'hydrogénophosphate disodique, 4,0 à 8,0 g/l de chlorure de sodium, 0,5 à 1,5 g/l de BSA, 0,25 à 0,5 g/l d'azoture de sodium et 0,25 à 0,5 g/l de Tween-20.

8. Kit de détection, comprenant : le complexe marqué selon l'une quelconque des revendications 1 à 3.

9. Kit de détection selon la revendication 8, comprenant en outre :
une solution de particules magnétiques recouverte d'un antigène ; ou
une solution de particules magnétiques recouverte de streptavidine et une solution d'antigène biotinylé ; ou
une solution de particules magnétiques recouverte d'un anticorps anti-isothiocyanate de fluorescéine et une solution d'antigène marquée à l'isothiocyanate de fluorescéine.

10. Kit de détection selon la revendication 9, dans lequel le kit de détection est un kit de détection d'un anticorps de VIH-1, comprenant le complexe marqué d'un antigène recombinant du VIH-1 ;
de préférence, le kit comprend spécifiquement :
une solution de particules magnétiques recouverte d'un antigène recombinant du VIH-1 et la solution complexe marquée de l'antigène recombinant du VIH-1 ; ou
une solution de particules magnétiques recouverte de streptavidine, la solution complexe marquée d'un antigène recombinant du VIH-1 et une solution d'antigène recombinant du VIH-1 biotinylé ; ou
une solution de particules magnétiques recouverte d'un anticorps anti-isothiocyanate de fluorescéine, une solution d'antigène recombinant du VIH-1 marquée à l'isothiocyanate de fluorescéine et la solution complexe marquée de l'antigène recombinant du VIH-1 ;
de préférence, une concentration de l'antigène recombinant du VIH-1 dans la solution de particules magnétiques recouverte de l'antigène recombinant du VIH-1 est de 10 à 200 pg/l ;
de préférence, la concentration des particules magnétiques est de 0,25 à 1,25 mg/ml ;
de préférence, une concentration de l'antigène recombinant du VIH-1 dans la solution complexe marquée de l'antigène recombinant du VIH-1 est de 10 à 200 pg/l ;
de préférence, une concentration du complexe marqué de l'antigène recombinant du VIH-1 est de 0,1 à 1 mg/l ;
de préférence, une concentration de l'antigène VIH-1 recombinant dans la solution d'antigène VIH-1 recombinant biotinylé est de 10 à 200 pg/l, et une concentration de biotine est de 0,1 à 1 mg/l ; et
de préférence, une concentration de l'antigène VIH-1 recombinant dans la solution d'antigène VIH-1 recombinant marquée à l'isothiocyanate de fluorescéine est de 10 à 200 pg/l, et une concentration de l'isothiocyanate de fluorescéine est de 0,1 à 1 mg/l.

11. Application du kit selon l'une quelconque des revendications 8 à 10 dans l'immunodétection, dans laquelle une protéine marqueur et une protéine détectée sont des protéines non homologues.

12. Application selon la revendication 11, dans laquelle le kit est utilisé pour détecter un anticorps de VIH-1, un anticorps de VIH-2, un virus de l'hépatite A, un virus de l'hépatite B, un virus de l'hépatite C, un virus de l'hépatite D, un virus de l'hépatite E, un virus de l'hépatite G, un virus des lymphocytes T humains, un anticorps de Treponema pallidum, un anticorps d'Helicobacter pylori ou un anticorps de virus du papillome humain ;
de préférence, le kit est appliqué à un analyseur immunitaire semi-automatique ou automatique.

13. Système de détection, comprenant : le kit selon l'une quelconque des revendications 8 à 10 et un analyseur immunitaire semi-automatique ou automatique.

## Patentansprüche

1. Markierter Komplex, umfassend:
ein Zwischenprodukt für einen markierten Komplex, wobei das Zwischenprodukt für einen markierten Komplex ein Antigen und ein Marker-Protein umfasst, wobei das Marker-Protein eine funktionelle Gruppe auf der Oberfläche davon aufweist und die funktionelle Gruppe eines oder mehrere von einer Carboxyl-, Amino-, Hydroxyl-, Sulfhydryl-, Aldehydgruppe, Glycosyl und Imidazolyl ist und die funktionelle Gruppe kovalent mit einer freien Amino-, Carboxyl-, Sulfhydryl-, Aldehydgruppe oder Glycosyl auf dem Antigen gekoppelt ist und wobei das Marker-Protein Meerrettichperoxidase, alkalische Phosphatase, saure Phosphatase, Glucoseoxidase oder β-Galactosidase ist; und
eine Signalerzeugungssubstanz, die kovalent mit dem Marker-Protein des Zwischenprodukts für einen markierten Komplex gekoppelt ist, um den markierten Komplex zu bilden;
wobei das Marker-Protein und die Signalerzeugungssubstanz unterschiedliche Substanzen sind.

2. Markierter Komplex nach Anspruch 1, wobei das Antigen ein rekombinantes Antigen oder ein natürliches Antigen ist;
bevorzugt das Antigen ein rekombinantes HIV-1-Antigen, ein rekombinantes HIV-2-Antigen, ein Hepatitis-A-Virusantigen, ein Hepatitis-B-Virusantigen, ein Hepatitis-C-Virusantigen, ein Hepatitis-D-Virusantigen, ein Hepatitis-E-Virusantigen, ein Hepatitis-G-Virusantigen, ein humanes T-Lymphozytvirusantigen, ein Treponema-pallidum-Antigen, ein Helicobacter-pylori-Antigen oder ein humanes Papillomvirusantigen ist und bevorzugt das Antigen das rekombinante HIV-1-Antigen oder das rekombinante HIV-2-Antigen ist.

3. Markierter Komplex nach einem der Ansprüche 1-2, wobei die Signalerzeugungssubstanz Luminol, Isoluminol, ein Isoluminolderivat, alkalische Phosphatase, Meerrettichperoxidase, eine fluoreszierende Substanz, ein Seltenerd-Ion, ein Seltenerd-Ion-Chelatligand, Acridiniumester, ein Acridiniumesterderivat oder Tris(bipyridin)ruthenium ist und bevorzugt das Isoluminolderivat N-(4-Aminobutyl)-N-ethylisoluminol ist.

4. Verfahren zum Zubereiten des markierten Komplexes nach einem der Ansprüche 1-3, umfassend die folgenden Schritte:
Durchführen einer kovalenten Kopplungsreaktion zwischen einem Marker-Protein, das eine funktionelle Gruppe auf der Oberfläche davon aufweist, und einem Antigen, um ein Zwischenprodukt für einen markierten Komplex zu erlangen; und
Durchführen einer kovalenten Kopplungsreaktion zwischen einer Signalerzeugungssubstanz und dem Marker-Protein des Zwischenprodukts für einen markierten Komplex, um den markierten Komplex zu erlangen;
wobei die funktionelle Gruppe eines oder mehrere von einer Carboxyl-, Amino-, Hydroxyl-, Sulfhydryl-, Aldehydgruppe, Glycosyl und Imidazolyl ist und die funktionelle Gruppe kovalent mit einer freien Amino-, Carboxyl-, Sulfhydryl-, Aldehydgruppe oder Glycosyl auf dem Antigen gekoppelt ist und
wobei das Marker-Protein Meerrettichperoxidase, alkalische Phosphatase, saure Phosphatase, Glucoseoxidase oder β-Galactosidase ist;
wobei das Marker-Protein und die Signalerzeugungssubstanz unterschiedliche Substanzen sind.

5. Verfahren nach Anspruch 4, wobei das Verfahren, das für die kovalente Kopplungsreaktion verwendet wird, ein gemischtes Anhydridverfahren, ein Carbodiimidverfahren, ein Glutaraldehydverfahren, ein Glutaranhydridverfahren, ein Diazotierungsverfahren, ein Bernsteinsäureverfahren, ein Carbonyldiimidazolverfahren oder ein Natriumperiodatverfahren ist,
und bevorzugt ein Vernetzungsmittel, das in der kovalenten Kopplungsreaktion verwendet wird, ein homobifunktionelles aminoreaktives Proteinvernetzungsmittel basierend auf NHS-Ester und einer imidoatreaktiven Gruppe, ein Sulfhydryl-Saccharidvernetzungsmittel basierend auf Maleimid und einer hydrazidreaktiven Gruppe, ein homobifunktionelles aminoreaktives Proteinvernetzungsmittel basierend auf Maleimid oder einer pyridindisulfhydrylreaktiven Gruppe, ein heterobifunktionelles Proteinvernetzungsmittel, das zum Verknüpfen eines Proteins mit einem primären Amin und einem Sulfhydryl eines anderen Moleküls geeignet ist, ein Carbodiimidvernetzungsmittel, das zum Verknüpfen von Carboxyl mit primärem Amin geeignet ist, ein heterobifunktionelles Vernetzungsmittel, das in fotoreaktiven Vernetzungsmitteln beinhaltet ist, ein Chemoselektivitätsvernetzungsmittel oder ein bifunktionelles Vernetzungsmittel ist.

6. Verfahren nach Anspruch 4, wobei ein Molverhältnis des Marker-Proteins zu dem Antigen bei Durchführen der kovalenten Kopplungsreaktion zwischen dem Marker-Protein und dem Antigen 1:1 ~ 3:1 beträgt;
bevorzugt eine Pufferlösung, die in der kovalenten Kopplungsreaktion verwendet wird, eine PBS-Pufferlösung, deren pH 4,0-6,5 beträgt, eine Carbonatpufferlösung, deren pH 8,0-9,8 beträgt, eine Glutaraldehydlösung, deren Konzentration 0,5 %-5 % beträgt und deren pH 7,0-8,0 beträgt, oder eine MES-Pufferlösung, deren pH 4,0-6,0 beträgt, ist.

7. Verfahren nach Anspruch 4, wobei das Zwischenprodukt für einen markierten Komplex oder der markierte Komplex nach Zubereitung zur Aufbewahrung in eine Stammlösung platziert wird und die Stammlösung Folgendes umfasst: 0,2-0,5 g/l Kaliumdihydrogenphosphat, 3,0-6,0 g/l Dinatriumhydrogenphosphat, 4,0-8,0 g/l Natriumchlorid, 0,5-1,5 g/l BSA, 0,25-0,5 g/l Natriumazid und 0,25-0,5 ml/1 Tween-20.

8. Detektionskit, umfassend: den markierten Komplex nach einem der Ansprüche 1-3.

9. Detektionskit nach Anspruch 8, ferner umfassend:
eine Magnetpartikellösung, die mit einem Antigen beschichtet ist; oder
eine Magnetpartikellösung, die mit Streptavidin und einer biotinylierten Antigenlösung beschichtet ist; oder
eine Magnetpartikellösung, die mit einem Antifluoresceinisothiocyanat-Antikörper und einer Antigenlösung, die mit Fluoresceinisothiocyanat markiert ist, beschichtet ist.

10. Detektionskit nach Anspruch 9, wobei das Detektionskit ein Kit zum Detektieren eines HIV-1-Antikörpers ist, das den markierten Komplex eines rekombinanten HIV-1-Antigens umfasst;
bevorzugt das Kit spezifisch Folgendes umfasst:
eine Magnetpartikellösung, die mit einem rekombinanten HIV-1-Antigen und der Lösung des markierten Komplexes des rekombinanten HIV-1-Antigens beschichtet ist; oder
eine Magnetpartikellösung, die mit Streptavidin, der Lösung des markierten Komplexes eines rekombinanten HIV-1-Antigens und einer biotinylierten rekombinanten HIV-1-Antigenlösung beschichtet ist; oder
eine Magnetpartikellösung, die mit einem Antifluoresceinisothiocyanat-Antikörper, einer rekombinanten HIV-1-Antigenlösung, die mit Fluoresceinisothiocyanat und der Lösung des markierten Komplexes des rekombinanten HIV-1-Antigens beschichtet ist;
bevorzugt eine Konzentration des rekombinanten HIV-1-Antigens in der Magnetpartikellösung, die mit dem rekombinanten HIV-1-Antigen beschichtet ist, 10-200 pg/l beträgt;
bevorzugt eine Konzentration der Magnetpartikel 0,25-1,25 mg/ml beträgt;
bevorzugt eine Konzentration des rekombinanten HIV-1-Antigens in der Lösung des markierten Komplexes des rekombinanten HIV-1-Antigens 10-200 pg/l beträgt;
bevorzugt eine Konzentration des markierten Komplexes des rekombinanten HIV-1-Antigens 0,1-1 mg/l beträgt;
bevorzugt eine Konzentration des rekombinanten HIV-1-Antigens in der biotinylierten rekombinanten HIV-1-Antigenlösung 10-200 pg/l beträgt und eine Konzentration von Biotin 0,1-1 mg/l beträgt; und
bevorzugt eine Konzentration des rekombinanten HIV-1-Antigens in der rekombinanten HIV-1-Antigenlösung, die mit Fluoresceinisothiocyanat markiert ist, 10-200 pg/l beträgt und eine Konzentration des Fluoresceinisothiocyanats 0,1-1 mg/l beträgt.

11. Anwendung des Kits nach einem der Ansprüche 8-10 bei Immunnachweis, wobei ein Marker-Protein und ein detektiertes Protein nicht-homologe Proteine sind.

12. Anwendung nach Anspruch 11, wobei das Kit zum Detektieren eines HIV-1-Antikörpers, eines HIV-2-Antikörpers, eines Hepatitis-A-Virus, eines Hepatitis-B-Virus, eines Hepatitis-C-Virus, eines Hepatitis-D-Virus, eines Hepatitis-E-Virus, eines Hepatitis-G-Virus, eines humanen T-Lymphozytvirus, eines Treponema-pallidum-Antikörpers, eines Helicobacter-pylori-Antikörpers oder eines humanen Papillomvirusantikörpers verwendet wird;
bevorzugt das Kit auf einen halbautomatischen oder automatischen Immunanalysator angewendet wird.

13. Detektionssystem, umfassend: das Kit nach einem der Ansprüche 8-10 und einen halbautomatischen oder automatischen Immunanalysator.
